# EUROPÄISCHE PATENTSCHRIFT

(11) **EP 0 626 159 B1**
(45) Veröffentlichungstag und Bekanntmachung des Hinweises auf die Patenterteilung: **14.07.1999**
(21) Anmeldenummer: 94106588.0
(22) Anmeldetag: 27.04.1994
(51) Int. Cl.: A61F 13/15

(54) **Decklage von Körperflüssigkeit absorbierenden Artikeln und Verfahren zur Herstellung derselben**
Coversheet for body fluid absorbing articles and method of production of the same
Couche supérieure pour articles absorbants des fluides du corps, et méthode pour sa fabrication

(30) Priorität: 27.04.1993 JP 10119093
(43) Veröffentlichungstag der Anmeldung: 30.11.1994
(73) Patentinhaber: UNI-CHARM CORPORATION, Kawanoe-shi Ehime-ken (JP)
(72) Erfinder: Takai, Hisashi, Kawanoe-shi, Ehime-ken (JP); Kido, Tsutomu, Kawanoe-shi, Ehime-ken (JP)
(74) Vertreter: Sperling, Rüdiger, Dipl.-Ing.

(56) Entgegenhaltungen:
- EP-A- 0 040 447
- EP-A- 0 235 309
- EP-A- 0 304 617
- US-A- 3 542 028

## Beschreibung

### TECHNISCHER HINTERGRUND DER ERFINDUNG

Die vorliegende Erfindung betrifft eine Decklage, die für Körperflüssigkeit absorbierende Artikel verwendet wird, wie z.B. Damenbinden und Wegwerfwindeln, sowie ein Verfahren zur Herstellung der Decklage.

Es ist bekannt, eine für Körperflüssigkeit absorbierende Artikel verwendete Decklage zu bilden und diese Decklage mit einer Vielzahl von Flüssigkeitskanälen zu versehen, die jeweils durch die Lage von einer oberen Öffnung zu einer unteren Öffnung verlaufen, so daß die Oberfläche mit der Haut eines Trägers in Berührung kommt und die unteren Öffnungen der Flüssigkeitskanäle in Berührung mit einem flüssigkeitsabsorbierenden Kern stehen und somit Körperflüssigkeiten in den flüssigkeitsabsorbierenden Kern unter einer innerhalb der jeweiligen Flüssigkeitskanäle auftretenden Kapillarwirkung eingeführt werden.

Beispielsweise zeigt die japanische Patentveröffentlichung Nr. 1982-17081 eine Technik auf, bei der ein aus einer Polyethylenfolie hergestellte Decklage mit jeweils in Form von kegelstumpfförmigen konischen Kapillarröhrchen ausgebildeten Flüssigkeitskanälen versehen ist und ein unteres Ende in enger Berührung mit einem flüssigkeitsabsorbierenden Kern angeordnet ist.

Die japanische Patentanmeldung Veröffentlichungsnummer 1992-58950 zeigt eine Technik auf, bei der thermoplastische Synthetikfasern schmelzgeblasen werden, um ein Vliesstoff zu bilden, der anschließend mit Flüssigkeitskanälen versehen wird und bei dem Faserflaum um die unteren Öffnungen der Flüssigkeitskanäle ausgebildet wird, um eine Decklage zu erhalten.

Bei beiden der vorstehend erwähnten bekannten Techniken sollte das untere Ende jedes Flüssigkeitskanales immer in engem Kontakt mit dem flüssigkeitsabsorbierenden Kern stehen. Die Decklage, die dünn und weniger starr ist, wird jedoch während der praktischen Verwendung von Körperflüssigkeit absorbierenden Artikeln leicht zerknittert und folglich wird das untere Ende des Flüssigkeitskanales vom flüssigkeitsabsorbierenden Kern getrennt, was einen problemlosen Übergang der Körperflüssigkeiten in den flüssigkeitsabsorbierenden Kern verhindert.

Allgemein ist ein weicher Griff für Körperflüssigkeit absorbierende Artikel unverzichtbar. Beispielsweise ist im Fall des aus Faserpulpe geformten flüssigkeitsabsorbierenden Kerns die Dichte desselben beschränkt, da eine übermäßig hohe Dichte der Faserpulpe dem Träger ein unangenehm starres Tragegefühl verleihen würde. Eine übermäßig geringe Dichte der Faserpulpe würde jedoch das Verteilungsvermögen der Körperflüssigkeiten verringern. Dadurch können sich die von den Flüssigkeitskanälen zu dem flüssigkeitsabsorbierenden Kern übergehenden Körperflüssigkeiten nicht rasch seitlich von den unteren Öffnungen verteilen und die Körperflüssigkeiten verbleiben für einen entsprechend längeren Zeitraum auf der Decklage, wodurch der Träger ein unangenehmes Gefühl der Feuchtigkeit hat.

Demgemäß ist es eine Hauptaufgabe der Erfindung, das bei den vorstehend erwähnten Techniken nach dem Stand der Technik auftretende Problem zu lösen, indem eine Decklage aus einer ersten Lage und einer zweiten Lage zusammengesetzt wird, die zweite Lage mit der ersten Lage um untere Öffnungen von jeweiligen durch die erste Lage ausgebildeten Flüssigkeitskanälen verbunden wird, und die Dichte der zweiten Lage auf ein Dichteniveau eines flüssigkeitsabsorbierenden Kernes eingestellt wird.

### KURZBESCHREIBUNG DER ERFINDUNG

Vorstehend beschriebene Aufgabe wird gemäß einem Aspekt der Erfindung durch die Merkmale des Anspruchs 1 gelöst.

Vorzugsweise hat die zweite Lage der Decklage in der Längsrichtung der Decklage ausgerichtete Fasern und die zweite Lage ist aus einer Mischung von 70 - 100 Gewichtsprozent Zellulosefasern und 30 - 0 Gewichtsprozent thermoplastischen Synthetikfasern zusammengesetzt.

Vorstehend dargelegte Aufgabe wird gemäß einem weiteren Aspekt der Erfindung durch ein Verfahren zur Herstellung einer in Körperflüssigkeit absorbierenden Artikeln verwendeten Decklage gemäß Anspruch 4 gelöst.

Vorzugsweise hat das Faservlies in Längsrichtung der Decklage ausgerichtete Fasern. Das Faservlies ist vorzugsweise aus einer Mischung von 70 - 100 Gewichtsprozent Zellulosefasern und 30 - 0 Gewichtsprozent thermoplastischen Synthetikfasern zusammengesetzt.

Bei der in vorstehend beschriebener Weise gemäß der Erfindung aufgebauten Decklage ist die zweite Lage um die unteren Öffnungen der jeweiligen Flüssigkeitskanäle mit der ersten Lage verbunden und dadurch mit der ersten Lage integriert. Somit ist die Steifigkeit der Decklage ausreichend verbessert, um ein Zerknittern der Decklage zu verhindern und die Verformung der unteren Öffnungen zu beschränken. Folglich werden die Flüssigkeitskanäle nicht ohne weiteres verstopft. Der Umfang jeder unteren Öffnung ist einerseits mit der zweiten Lage verbunden, die eine höhere Dichte als die Dichte der im flüssigkeitsabsorbierenden Kern enthaltenen Fasern hat, und die zweite Lage steht andererseits mit dem flüssigkeitsabsorbierenden Kern in Berührung, so daß die Körperflüssigkeiten, die die unteren Öffnungen der jeweiligen Flüssigkeitskanäle erreicht haben, nicht nur problemlos unmittelbar nach unten durch die zweite Lage in den flüssigkeitsabsorbierenden Kern übertreten können, sondern auch rasch durch die zweite Lage in der Oberflächenrichtung verteilt und anschließend in den flüssigkeitsabsorbierenden Kern übertragen werden können.

Die Fasern der zweiten Lage sind in einer gewünschten Richtung ausgerichtet und die Körperflüssigkeiten können problemlos insbesondere in der der Faserausrichtung entsprechenden Richtung verteilt werden. Beispielsweise kann die Faserausrichtung so gewählt werden, daß sie mit der Längsrichtung der Körperflüssigkeit absorbierenden Waren zusammenfällt, um sicherzustellen, daß die Körperflüssigkeiten rasch in Längsrichtung des flüssigkeitsabsorbierenden Kernes verteilt werden und der gesamte flüssigkeitsabsorbierende Kern effizient genutzt werden kann.

Entsprechend dem erfindungsgemäßen Verfahren zur Herstellung der Decklage können die jeweils von den vertieften Umfangsbereichen begrenzten Öffnungen durch Schmelzen der auf dem schmelzgeblasenen Vliesstoff gebildeten Vorsprünge gebildet werden und das Faservlies wird durch mechanisch verschlungene Fasern gebildet, so daß die Fasern dieses Vlieses problemlos entwirrt und zuverlässig um diese Umfangsbereiche verschlungen werden können. Dies hat zur Folge, daß der schmelzgeblasene Vliesstoff ohne weiteres mit dem Faservlies zu einem Stück verbunden werden kann.

### KURZBESCHREIBUNG DER ZEICHNUNGEN

Eine erfindungsgemäße Decklage und ein Verfahren zur Herstellung derselben werden im Detail unter Bezug auf die beiliegende Zeichnung beschrieben, wobei
Figur 1 eine perspektivische Darstellung einer Damenbinde ist;
Figur 2 eine Teilschnittdarstellung entlang einer Linie X-X in Figur 1 ist; und
Figur 3 eine schematische Darstellung einer Produktionslinie zur Bildung der Decklage zeigt.

### BEVORZUGTE AUSFÜHRUNGSFORMEN DER ERFINDUNG

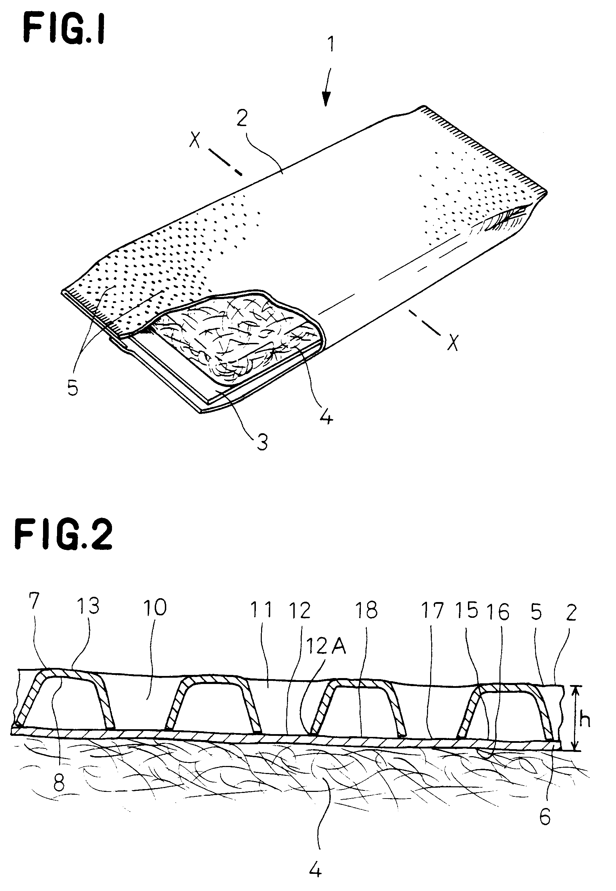

In Figur 1 ist eine Damenbinde 1 teilweise ausgebrochen dargestellt, bei der eine erfindungsgemäße Decklage 2 verwendet wird. Die Binde 1 umfaßt die flüssigkeitsdurchlässige Decklage 2, eine flüssigkeitsundurchlässige untere Lage 3 und eine zwischen diese Lagen 2,3 gelegten flüssigkeitsabsorbierenden Kern 4. Die Decklage 2 umhüllt den flüssigkeitsabsorbierenden Kern 4 vollständig, wobei die entgegengesetzten Seitenränder derselben an der Rückseite der Binde 1 miteinander verbunden sind und die oberen und unteren Schichten der in Längsrichtung entgegengesetzten Enden derselben entlang in Längsrichtung entgegengesetzten Enden der Binde 1 jeweils miteinander verbunden sind. Die untere Lage 3 ist zwischen die Decklage 2 und den flüssigkeitsabsorbierenden Kern auf der Rückseite der Binde 1 gelegt.

Figur 2 ist eine Teilschnittdarstellung entlang einer Linie X-X in Figur 1, die schematisch den Zustand darstellt, in dem die Decklage 2 mit dem flüssigkeitsabsorbierenden Kern 4 in Berührung steht. Die Decklage 2 umfaßt eine auf der Seite der Haut eines Trägers liegende obere Lage 5 und eine auf der Seite des flüssigkeitsabsorbierenden Kernes 4 liegende untere Lage 6. Die obere Lage 5 hat eine Oberfläche 7, eine Unterfläche 8, sich jeweils von der Oberfläche 7 zur Unterfläche 8 über eine Höhe h erstreckende Flüssigkeitskanäle 10 und eine sich kontinuierlich um die oberen Öffnungen 11 der jeweiligen Flüssigkeitskanäle 10 erstreckende Hautberührungsfläche 13. Die untere Lage 6 hat eine Oberfläche 15, eine Unterfläche 16 und eine freiliegende Fläche 17 innerhalb der jeweiligen Flüssigkeitskanäle 10 sowie eine nicht freiliegende Fläche 18, die der Hautberührungsfläche 13 gegenüberliegend angeordnet ist. Die Unterfläche 16 ist wenigstens im Bereich der freiliegenden Fläche 17 eben oder rundet sich nach außen zum flüssigkeitsabsorbierenden Kern 4, d.h. steigt nicht in die jeweiligen Flüssigkeitskanäle 10 an, so daß die Unterfläche 16 problemlos in dichte Berührung mit dem flüssigkeitsabsorbierenden Kern 4 gebracht wird. Im Bereich der nicht berührenden Fläche 18 ist die Unterfläche 16 vorzugsweise ähnlich der freiliegenden Fläche 17.

In der oberen Lage 5 ist der Umfang 12A der unteren Öffnungen 12 des Flüssigkeitskanales 10 durch eine Vielzahl von Unregelmäßigkeiten und Faserflaum vertieft. Andererseits umfaßt die untere Lage 6 ein durch mechanisch miteinander verschlungene Fasern gebildetes Vlies, die ebenfalls mit dem Umfangsbereich verschlungen sind, um so mit der oberen Lage 5 zu einem Stück verbunden zu werden und somit die Decklage 2 zu bilden. Die obere Lage 5 ist aus einer thermoplastischen Kunststoffolie oder einem Vliesstoff aus thermoplastischen und hydrophoben Synthetikfasern gebildet. Die Kunststoffolie kann beispielsweise eine Polyethylenfolie mit einer Dicke von 0,01-0,10 mm sein und der Vliesstoff kann beispielsweise schmelzgeblasener Vliesstoff mit einem Gewicht pro Flächeneinheit von 5 - 100 g/m² sein.

Die untere Lage 6 wird durch ein Faservlies gebildet, das 70 - 100 Gewichtsprozent Zellulosefasern, wie etwa Pulpe oder Rayon umfaßt, gemischt mit 30 - 0 Gewichtspozent thermoplastischer Synthetikfasern. Die untere Lage 6 ist hydrophiler als die obere Lage 5 und hat eine höhere Dichte als die Dichte der Fasern, die den flüssigkeitsabsorbierenden Kern 4 bilden. Wenn der flüssigkeitsabsorbierende Kern 4 beispielsweise aus einer Mischung von Faserpulpe und hochabsorbierenden Polymerpulvern gebildet wird, ist es bevorzugt, die Dichte der unteren Lage 6 auf ein höheres Niveau als die Dichte der Faserpulpe einzustellen. Gewöhnlich wird die Dichte derartiger Faserpulpe in einem Bereich von 0,02 - 0,1 g/cm³ gewählt. Viele der die untere Lage 6 bildenden Fasern haben eine in Längsrichtung der Binde 1 verlaufende Ausrichtung und Körperflüssigkeiten verteilen sich leicht durch Kapillarwirkung in der dieser Ausrichtungen entsprechenden Richtung. Falls gewünscht, kann der im wesentlichen aus Zellulosefasern zusammengesetzten unteren Lage 6 durch Zugabe oder Beimischung von 30 Gewichtsprozent oder weniger thermoplastischer Synthetikfasern ein höheres Verteilungsvermögen von Körperflüssigkeiten verliehen werden als das durch eine aus 100 Gewichtsprozent Zellulosefasern gebildete untere Lage erzielte Verteilungsvermögen.

Jeder Flüssigkeitskanal 10 der oberen Lage 5 kann ein konisch auf den flüssigkeitsabsorbierenden Kern 4 zulaufendes Kapillarrohr sein, ein in umgekehrter Richtung konisch ausgebildetes Kapillarrohr oder ein zylindrisches Kapillarrohr mit gleichförmigen Durchmesser. Der Flüssigkeitskanal 10 hat vorzugsweise eine Länge von 0,1 - 7 mm. Die unteren und oberen Öffnungen 11, 12 können kreisförmig, vieleckig oder in jeder anderen geeigneten Form ausgebildet sein und ihr Durchmesser beträgt vorzugsweise 0,3 - 7 mm.

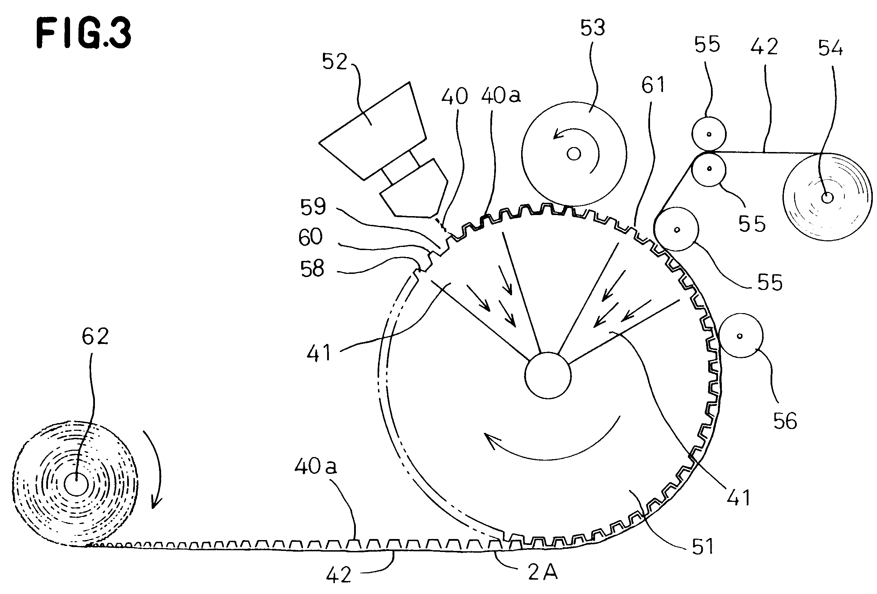

In Figur 3 ist eine Produktionslinie zur Bildung der Decklage 2 schematisch dargestellt. Diese Produktionslinie umfaßt eine im Uhrzeigersinn drehbare Walze 51, die als Form dient, einen der Walze 51 gegenüberliegend angeordneten Schmelzblasextruder 52, eine auf eine gewünschte Temperatur eingestellte Heizwalze 53, eine Faservlieszuliefereinrichtung 54 und eine Aufwickeleinrichtung 62.

Auf ihrer außeren Umfangsfläche ist die Walze 51 mit einer Vielzahl von Vorsprüngen 58 mit einer im wesentlichen gleichmäßigen Höhe versehen, die jeweils eine flache Oberseite aufweisen, sowie mit Vertiefungen 59 um die jeweiligen Vorsprünge 58. Der gesamte Umfang der Walze 51 ist mit einer Vielzahl von kleinen Durchgangslöchern (nicht dargestellt) versehen, die mit einer innerhalb der Walze 51 vorgesehenen Saugeinrichtung 41 in Verbindung stehen.

Geschmolzene Fasern 80 werden aus dem Schmelzblasextruder 52 auf die äußere Umfangsfläche der Walze 51 unter Einwirkung der Saugeinrichtung 41 aufgeblasen, um einen schmelzgeblasenen Vliesstoff 40a zu bilden, der den Vorsprüngen 58 und den Vertiefungen 59 folgt.

Die Heizwalze 53 ist so angeordnet, daß sie mit den flachen Oberseiten 60 der jeweiligen Vorsprünge 58 in Berührung kommt und den Vliesstoff 40a schmelzt, der zwischen der Heizwalze 53 und den flachen Oberseiten auf den jeweiligen flachen Oberseiten 60 liegt, um so Öffnungen 61 auszubilden.

Die Faservlieszuliefereinrichtung 54 führt ein Vlies 42 durch zwei Führungswalzen 55 auf den Vliesstoff 40a zu, der unter der Einwirkung der Saugeinrichtung 41 mit den Öffnungen 61 versehen wurde, und gleichzeitig wird das Vlies 42 mit dem schmelzgeblasenen Vliesstoff um die jeweiligen Öffnungen 61 unter der Wirkung einer Druckwalze 56 verschlungen. Der schmelzgeblasene Vliesstoff 40a und die Bahn 42, die auf diese Weise zu einem Stück verbunden sind, werden von der Aufwickeleinrichtung 62 aufgespult, um eine ungeschnittene Rolle 2A für die Decklagen 2 zu erhalten. Diese ungeschnittene Rolle 2A wird in einzelne, zur Verwendung für die einzelnen Binden 1 entsprechend dimensionierte Lagen geschnitten.

Der Aufbau der mit dem vorstehend beschriebenen Verfahren hergestellten ungeschnittenen Rolle 2A ist mit dem Aufbau der einzelnen Decklage 2 verwandt, die aus der ungeschnittenen Rolle 2A geschnitten wird, so daß der schmelzgeblasene Vliesstoff 40a und das Vlies 42 die obere bzw. die untere Lage 5 bzw. 6 bilden, die durch die Vorsprünge und Vertiefungen verformten Abschnitte die Flüssigkeitskanäle bzw. die Hautberührungsfläche 13 bilden, und die Abschnitte des Vlieses, die über den jeweiligen Oberseiten 60 der Vorsprünge liegen, die freiliegende Fläche 17 bilden. Die freiliegende Fläche 17 erhebt sich nicht in Richtung der oberen Öffnung 11 in der Decklage 2 und wird zuverlässig mit dem flüssigkeitsabsorbierenden Kern 4 bei Verwendung in der Binde 1 in Berührung gebracht, da die Oberseiten 60 flach sind.

Das Faservlies 42 wird durch ein nur mechanisch verschlungene Faser umfassendes Vlies, wie z.B. Wirrfaservlies, Kardenvlies, Blasvlies, oder Vliesstoff gebildet, das erhalten wird, indem einer dieser Vliesstoffe der Verschlingung unter einem Hochdruckwasserstrahl unterzogen wird und auf eine Dichte eingestellt wird, die höher ist als die Dichte der in dem tatsächlich verwendeten flüssigkeitsabsorbierenden Kern 4 enthaltenen Fasern. Das Faservlies 42 enthält hydrophile Zellulosefasern, wie etwa Pulpe oder Rayon, die 70 - 100 Gewichtsprozent hinsichtlich einer Gesamtmenge der Fasern einnehmen, und, falls gewünscht, können derartige hydrophile Fasern mit hydrophoben thermoplastischen Synthetikfasern zu 30 - 0 Gewichtsprozent gemischt werden. Die Fasern eines derartigen Vlieses oder eines derartigen Vliessstoffes sind gewöhnlich in Bearbeitungsrichtung während ihres Herstellungsablaufes ausgerichtet und daher wird das Vlies 42 so zugeliefert und/oder geschnitten, daß die Faserausrichtung mit der Längsrichtung der herzustellenden Binde 1 zusammenfällt.

Die erfindungsgemäße Decklage ermöglicht es, die Verformung der Flüssigkeitskanäle in wirksamer Weise einzuschränken, indem die untere Lage mit der oberen Lage verbunden wird.

Die untere Lage ist so eingestellt, daß sie eine höhere Dichte als die Dichte des flüssigkeitsabsorbierenden Kernes aufweist und folglich dringen die Körperflüssigkeiten, nachdem sie das untere Ende eines jeweiligen Flüssigkeitskanales erreicht haben, nicht nur unmittelbar durch die untere Lage in den flüssigkeitsabsorbierenden Kern, sondern verteilen sich auch durch die untere Lage in der Oberflächenrichtung und durchdringen anschließend diese nach unten in den flüssigkeitsabsorbierenden Kern. Auf diese Weise werden bei Körperflüssigkeit absorbierenden Artikeln, für die die erfindungsgemäße Decklage verwendet wird, die Körperflüssigkeiten in den flüssigkeitsabsorbierenden Kern über eine relativ große Fläche absorbiert und ein Zeitraum, in dem die Körperflüssigkeiten auf der Decklage bleiben, wird entsprechend verkürzt. Folglich wird das unangenehme Gefühl von Feuchtigkeit gemildert.

Die Fasern der unteren Lage können in der gewünschten Richtung ausgerichtet sein, um die Verteilung der Körperflüssigkeiten in dieser Richtung zu beschleunigen, und diese Verteilung kann weiter durch Beimischen der thermoplastischen Synthetikfasern in die untere Lage gefördert werden.

Das nur mechanisch verschlungene Fasern umfassende Vlies wird als untere Lage verwendet, so daß die Fasern problemlos entwirrt und leicht um die vertieften Umfangsbereiche jeder unteren Öffnung der jeweiligen Flüssigkeitskanäle verschlungen werden. Dieses Merkmal erleichtert die Verbindung der oberen und der unteren Lage zur Decklage.

## Patentansprüche

1. Absorbierender Artikel für Körperflüssigkeit mit einer flüssigkeitsdurchlässigen Decklage (2), die eine Vielzahl von jeweils durch diese von einer oberen Öffnung (11) zu einer unteren Öffnung (12) verlaufenden Flüssigkeitskanälen (10) und eine Hautberührungsfläche (13) aufweist, die sich kontinuierlich um die oberen Öffnungen (11) der Flüssigkeitskanäle (10) erstreckt, mit einer flüssigkeitsundurchlässigen unteren Lage (3) und einem zwischen diese Lagen gelegten flüssigkeitsabsorbierenden Kern (4), wobei die Decklage
- eine erste, aus einer thermoplastischen Kunststofflage hergestellte und mit den Flüssigkeitskanälen (10) und der Hautberührungsfläche (13) versehene Lage (5) und
- eine unter der ersten Lage liegende und mit dieser um die unteren Öffnungen der jeweiligen Flüssigkeitskanäle verbundene zweite Lage (6) umfaßt,
- wobei die zweite Lage (6) nur aus mechanisch verschlungenen Fasern hergestellt ist und eine höhere Dichte als die Dichte von in dem flüssigkeitsabsorbierenden Kern (4) enthaltenen Fasern aufweist.

2. Absorbierender Artikel nach Anspruch 1, wobei die Fasern der zweiten Lage (6) in Längsrichtung der Decklage (2) ausgerichtet sind.

3. Absorbierender Artikel nach Anspruch 1 oder 2, wobei die zweite Lage (6) eine Mischung von 70-100 Gew.-% Zellulosefasern und 30-0 Gew.-% thermoplastische Synthetikfasern umfaßt.

4. Verfahren zur Herstellung einer Decklage (2), die in Körperflüssigkeit absorbierenden Artikeln verwendbar ist, die eine Vielzahl von jeweils durch diese von einer oberen Öffnung (11) zu einer unteren Öffnung (12) verlaufenden Flüssigkeitskanälen (10) und eine sich kontinuierlich um die oberen Öffnungen der Flüssigkeitskanäle erstreckende Hautberührungsfläche (13) aufweist, wobei der Artikel eine flüssigkeitsundurchlässige untere Lage (13) und einen zwischen der Decklage und der unteren Lage gelegten flüssigkeitsabsorbierenden Kern (4) aufweist, wobei das Verfahren die Schritte umfaßt:
- Aufblasen von geschmolzenen Fasern (80) aus einem Schmelzblasextruder (52) auf eine Form mit einer Vielzahl von Vorsprüngen, die im wesentlichen in gleichförmiger Höhe dimensioniert sind, wobei die Vorsprünge flache Oberseiten haben, um einen schmelzgeblasenen Vliesstoff entsprechend der Konfiguration der Form zu bilden;
- Berühren des schmelzgeblasenen Vliesstoffes an den Oberseiten mit einem Heizelement (53) um Öffnungen zu bilden, die jeweils von einem vertieften Umfangsbereich begrenzt sind; und
- Zuführen eines nur mechanisch verschlungene Fasern umfassenden Faservlieses (42) mit einer Dichte, die höher ist als die Dichte von in dem flüssigkeitsabsorbierenden Kern enthaltenen Fasern, auf den schmelzgeblasenen Vliesstoff und dadurch mechanisches Verschlingen des Faservlieses um die vertieften Umfangsbereiche, um die Decklage zu erhalten.

5. Verfahren nach Anspruch 4, wobei die Fasern des Faservlieses (42) in Längsrichtung ausgerichtet sind.

6. Verfahren nach Anspruch 4, wobei das Faservlies eine Mischung von 70-100 Gew.-% Zellulosefasern und 30-0 Gew-% thermoplastische Synthetikfasern umfaßt.

## Claims

1. Absorbing article for body fluid with a liquid-permeable coversheet (2) which has a multiplicity of liquid channels (10) extending respectively through the said coversheet (2) from one upper opening (11) to a lower opening (12) and a skin contact surface (13) which extends continuously around the upper openings (11) of the liquid channels (10), with a liquid-permeable lower layer (3) and a liquid-absorbing core (4) lying between these layers, the coversheet comprising
a first layer (5) which is produced from a thermoplastic plastic material and which is provided with liquid channels (10) and the skin contact surface (13) and
a second layer (6) which lies under the first layer and is connected with the latter around the lower openings of the liquid channels respectively,
the second layer (6) being produced only from mechanically interlaced fibres and having a higher density than the density of the fibres contained in the liquid-absorbing core (4).

2. Absorbing article according to claim 1 in which the fibres of the second layer (6) are orientated in the longitudinal direction of the coversheet (2).

3. Absorbing article according to claim 1 or 2 in which the second layer (6) comprises a mixture of 70 - 100% by weight of cellulose fibres and 30 - 0 % by weight of thermoplastic synthetic fibres.

4. Method for producing a coversheet (2), which can be used in articles for absorbing body fluid, which has a multiplicity of liquid channels (10) extending respectively through said coversheet from an upper opening (11) to a lower opening (12) and which has a skin contact surface (13) extending continuously around the upper openings of the liquid channels, the article having a liquid-permeable lower layer (13) and a liquid-absorbing core (4) lying between the coversheet and the lower layer, the method comprising the steps:
inflation of molten fibres (80) from a liquid-blowing extruder (52) into a form with a multiplicity of projections which are dimensioned essentially uniform in height, the projections having flat upper-sides in order to form a liquid-blown bonded fabric corresponding to the configuration of the form;
contact of the liquid-blown bonded fabric on the upper-sides with a heating element (53) to form openings which are delimited respectively by a recessed periphery region; and
supply of a fabric fleece (42), comprising only mechanically interlaced fibres with a density which is greater than the density of the fibres contained in the liquid-absorbing core, onto the liquid-blown bonded fabric and consequently mechanical interlacing of the fibre fleece around the recessed periphery regions to maintain the coversheet.

5. Method according to claim 4 in which the fibres of the fibre fleece (42) are orientated in a longitudinal direction.

6. Method according to claim 4 in which the fibre fleece comprises a mixture of 70 - 100 % by weight of cellulose fibres and 30 - 0 % by weight of thermoplastic synthetic fibres.

## Revendications

1. Article absorbant pour fluides corporels, comprenant une couche de revêtement (2) perméable aux liquides, comportant une multitude de passages à liquide (10) traversant cette dernière depuis un orifice supérieur (11) en direction d'un orifice inférieur (12), et une face (13) en contact avec la peau qui s'étend en continu autour des orifices supérieurs (11) des passages à liquide (10), une couche inférieure (3) imperméable aux liquides et un corps central (4) absorbant les liquides, placé entre ces couches, article dans lequel ladite couche de revêtement
- comprend une première épaisseur (5) faite d'une couche synthétique en matière thermoplastique et pourvue des passages à liquide (10) et de la face (13) en contact avec la peau, et
- une seconde épaisseur (6) sous-jacente à la première épaisseur et raccordée à cette dernière autour des orifices des passages à liquide (10),
- ladite seconde épaisseur (6) n'étant constituée que de fibres enchevêtrées mécaniquement, et présentant une densité supérieure à la densité des fibres contenues dans le corps central absorbant les liquides (4).

2. Article absorbant selon la revendication 1, dans lequel les fibres de la seconde épaisseur (6) sont orientées dans la direction longitudinale de la couche de revêtement (2).

3. Article absorbant selon l'une des revendications 1 ou 2, dans lequel la seconde épaisseur (6) comporte un mélange de 70 à 100 % en poids de fibres de cellulose, et de 30 à 0 % en poids de fibres synthétiques thermoplastiques.

4. Procédé pour confectionner une couche de revêtement (2) utilisable dans des articles absorbant les fluides corporels, qui présente une multitude de passages à liquide (10) traversant cette dernière depuis un orifice supérieur (11) en direction d'un orifice inférieur (12) et une face (13) en contact avec la peau s'étendant en continu autour des orifices des passages à liquide, ledit article présentant une couche inférieure (3) imperméable aux liquides et un corps central (4) absorbant les liquides, disposé entre la couche de revêtement et ladite couche inférieure, le procédé comportant les étapes consistant à :
- provoquer l'expansion de fibres fondues (80) dans une extrudeuse de soufflage en fusion (52) sur une forme présentant une multiplicité de salles de dimensions sensiblement uniformes en hauteur, les saillies présentant des faces supérieures plates, pour obtenir un non tissé soufflé en fusion en accord avec la configuration de la forme;
- contacter le non tissé soufflé en fusion sur ses faces supérieures, avec un élément chauffant (53), pour former des orifices respectivement délimités par une zone périphérique approfondie; et
- amener un voile de fibres (42) d'une densité supérieure à la densité des fibres contenues dans le corps central absorbant les liquides, sur le non tissé soufflé en fusion, et provoquer ainsi l'enchevêtrement mécanique du voile de fibres autour des zones périphériques approfondies, pour obtenir la couche de revêtement.

5. Procédé selon la revendication 4, dans lequel les fibres du voile de fibres (42) sont orientées longitudinalement.

6. Procédé selon la revendication 4, dans lequel le voile de fibres comporte un mélange de 70 à 100% en poids de fibres de cellulose et de 30 à 0% en poids de fibres synthétiques thermoplastiques.
